# EUROPEAN PATENT SPECIFICATION

(11) **EP 3 471 674 B1**
(45) Date of publication and mention of the grant of the patent: **18.03.2020**
(21) Application number: 17762211.5
(22) Date of filing: 31.07.2017
(51) Int. Cl.: A61F 9/007

(54) **SUBRETINAL FLUID DRAINAGE INSTRUMENTS**
INSTRUMENTE ZUR SUBRETINALEN FLÜSSIGKEITSDRAINAGE
INSTRUMENTS DE DRAINAGE DE FLUIDE SOUS-RÉTINIEN

(30) Priority: 10.08.2016 US 201615233711
(43) Date of publication of application: 24.04.2019
(73) Proprietor: Alcon Inc., 1701 Fribourg (CH)
(72) Inventor: CHARLES, Steven, T., Germantown Tennessee 38138 (US)
(74) Representative: Hanna Moore + Curley
(86) International application number: PCT/IB2017/054665
(87) International publication number: WO 2018/029570

(56) References cited:
- WO-A1-2016/019160
- US-A- 4 530 356
- US-A1- 2014 171 997
- US-A1- 2015 173 947

## Description

### TECHNICAL FIELD

The present disclosure is directed to instruments, systems, and methods for draining fluid from behind a retinal membrane of an eye.

### BACKGROUND

Under normal conditions in the human eye, the retina is physically attached to the choroid. Vitreous humor, a transparent jellylike material, fills the posterior segment of the eye and also helps secure the retina against the choroid.

Some ophthalmic conditions are characterized by detachment of the retina from the retinal pigment epithelium (RPE) and choroid. This typically happens when there is a tear in the retina. Retinal tears may allow vitreous humor or aqueous humor to flow between the retina and the RPE/choroid, resulting in an undesirable buildup of subretinal fluid. This may detach a portion of the retina from the choroid. However, when the retina detaches from the RPE/choroid, the detached portion of the retina is no longer able to receive nourishment from the choroid, which may cause the detached portions of the retina to be permanently damaged, resulting in loss of vision.

Repairing these conditions typically requires a surgical intervention. A surgeon may insert a probe or other instrument into the posterior segment of the eye via a sclerotomy, an incision through the sclera at the pars plana. While viewing the posterior segment under a microscope, the surgeon may cut and aspirate vitreous using a vitrectomy probe in order to gain access to the retinal detachment or tear. The surgeon may manipulate and flatten the detached or torn portion of the retina against the RPE/choroid in its proper location. An additional fluid, such as a gas or a silicone oil can be injected into the eye to serve as a retinal tamponade fluid to maintain the detached portion of the retina against the RPE/choroid.

The surgeon then typically drains any subretinal fluid present between the retina and the choroid through a retinal break or a purpose-made retinotomy and then initiates fluid air exchange while continuing drainage of subretinal fluid. After the detached or torn portion of the retina is properly located and the subretinal fluid is drained, the surgeon may take additional steps to secure the retina in place typically by applying laser energy to the retinal defects.

Reference is made to the US 2015/173947 and to WO 2016/019160 which have been cited as relating to the background art.

### SUMMARY

The present disclosure is directed to instruments, systems, and methods of removing unwanted material from subretinal space in the eye.

It will be appreciated that the scope of the invention is in accordance with the claims. Accordingly, there is provided a surgical instrument as defined in claim 1. Further optional features are provided in accordance with the dependent claims.

Exemplary medical instruments and systems are provided herein. An exemplary surgical instrument for removal of subretinal fluid may include a handle coupleable to an aspiration pressure source. The handle may have a rotational structure rotatable around an axis of a handle body when manipulated by a user. The surgical instrument may include a first elongate tubular member having a first proximal end and a first distal end and may include a second elongate tubular member having a second proximal end and a second distal end. The first proximal end of the first elongate tubular member may be coupled to the rotational structure such that the first elongate tubular member is rotatable around the axis of the handle body. The second proximal end of the second elongate tubular member may be coupled to the first distal end of the first elongate tubular member and may be curved when exposed to body temperature. The surgical instrument may further include a port formed through a wall of the second elongate tubular member for aspirating material from a body cavity through the first and second elongate tubular members.

Another exemplary surgical instrument may include a handle that may be coupled to a conduit. The handle may have a rotational structure rotatable around an axis of a handle body of the handle. The surgical instrument may further include a first elongate tubular member having a first proximal end; a first distal end; and a first lumen extending through the first elongate tubular member. The first proximal end may be coupled to the rotational structure such that the first elongate tubular member is rotatable around the axis. A second elongate tubular member may also be included in the surgical instrument and may have a second proximal end; a second distal end; and a second lumen extending through the second elongate tubular member. The first lumen may be in fluid communication with the second lumen. The second proximal end of the second elongate tubular member may be coupled to the first distal end of the first elongate tubular member. The second distal end may be a rounded, closed distal tip. The surgical instrument may also include a port formed on one side of the second elongate tubular member and through a wall of the second elongate tubular member. The port may be operable to aspirate material from a body cavity through the first lumen and the second lumen. The body cavity may be a subretinal space. The port may occupy less than 90 degrees of the circumference of the second elongate tubular member.

An exemplary ophthalmic surgical system may include an aspiration pressure source coupled to a conduit and a surgical handpiece coupled to the aspiration pressure source by the conduit. The surgical handpiece may include a handle body coupled to the conduit. The handle body may have a rotational structure rotatable around an axis of the handle body. The surgical handpiece may further include a first elongate tubular member having a first proximal end; a first distal end; and a first lumen extending through the first elongate tubular member. The surgical handpiece may also include a second elongate tubular member having a second proximal end; a second distal end forming a closed distal tip; and a second lumen extending through the second elongate tubular member and in fluid communication with the first lumen. The first proximal end may be coupled to the rotational structure such that the first elongate tubular member is rotatable around the axis, and the second proximal end of the second elongate tubular member may be coupled to the first distal end of the first elongate tubular member. The second elongate tubular member may be more flexible than the first elongate tubular member. The surgical handpiece may also include a port formed through a wall of the second elongate tubular member for aspirating material from a body cavity through the first lumen and the second lumen. The second elongate tubular member may be curved. The port may be elliptical. The second elongate tubular member may be formed from a shape memory alloy.

Exemplary methods of cleaning a tissue surface are provided. An exemplary method may include introducing a surgical instrument, such as an implementation of the exemplary surgical instruments described herein, into the eye of a patient. A distal region of the instrument may be positioned into subretinal space by a user. An aspiration pressure source may be activated to cause fluid to exit the subretinal space. A distal region of the instrument may be rotated by user manipulation of a rotational structure on a handle body of the instrument. A portion of the instrument outside the eye may be manipulated so that a second elongate tubular member of the instrument travels along the retina while the pressure gradient is used to aspirate material from the eye. As a result, the port of the second elongate tubular member travels along the retinal surface while the pressure gradient is used to aspirate material from the eye.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the accompanying drawings and the following detailed description.

### BRIEF DESCRIPTION OF THE DRAWINGS

The accompanying drawings illustrate implementations of the instruments, systems, and methods disclosed herein and together with the description, serve to explain the principles of the present disclosure.
FIG. 1 illustrates a perspective view of an exemplary surgical system.
FIG. 2 is an illustration of an exemplary block diagram of the exemplary surgical system of FIG. 1.
FIG. 3 is an illustration of an exemplary surgical instrument *in situ* in an eye.
FIGS. 4A and 4B illustrate the exemplary surgical instrument of FIG. 3 in two different states.
FIGS. 5A and 5B illustrate rotational capabilities of the exemplary surgical instrument of FIG. 3.
FIG. 6 is a flowchart of an example method for using the surgical instrument of FIG. 3 to clean the surface of a membrane.
FIG. 7 shows a distal end of an elongate tubular member of the example surgical instrument of FIG. 3 oriented relative to an eye and, particularly, to the retina of the eye.

The accompanying drawings may be better understood by reference to the following detailed description.

### DETAILED DESCRIPTION

For the purposes of promoting an understanding of the principles of the present disclosure, reference will now be made to the implementations illustrated in the drawings. Specific language will be used to describe the same. It will nevertheless be understood that no limitation of the scope of the disclosure is intended. Any alterations and further modifications to the described instruments, systems, methods, and any further application of the principles of the present disclosure are fully contemplated as would normally occur to one skilled in the art to which the disclosure relates. In particular, it is fully contemplated that the features, components, and/or steps described with respect to one implementation may be combined with the features, components, and/or steps described with respect to other implementations of the present disclosure. For example, although explanatory references are made to ophthalmic applications, other medical applications are included within the scope of the present disclosure. For simplicity, in some instances the same reference numbers are used throughout the drawings to refer to the same or like parts.

The present disclosure is directed to instruments, systems, and methods for removing subretinal fluid in order to reattach a torn or detached retina. The instruments and systems may include instruments having specially formed distal regions. In some examples, the distal region may be curved in a manner corresponding to the curvature of the eye. Some distal region implementations may include a side port on a single side of the distal region. Having the port, or in some implementations multiple ports, on a single side may prevent incarceration of the retina or other tissues and may simplify use of the instruments. A pressure gradient may cause the subretinal fluid to flow into the single side port for removal from the eye. Depending on the implementation, the pressure gradient may be naturally occurring, such as when pressure present in the eye (e.g., the intraocular pressure) is greater than pressure present within the instrument, thereby causing subretinal fluid to move toward the lower pressure, out of the eye. In additional implementations, the pressure gradient may be artificially induced by any aspiration pressure source, such as a pump, a vacuum, or other pressure gradient inducing device.

The single-side port of the disclosed instruments may permit the distal region to rotate within the eye while minimizing the chance of incarcerating the retina within the instrument. In contrast, when multiple ports are provided on an instrument, such as two ports that are laterally opposite each other, the instrument can grasp the retina and retinal pigment epithelium (RPE) as the instrument is rotated. This can cause further damage to the delicate tissues in the eye.

Some of the instruments of the present disclosure may have a distal region formed from a shape memory alloy, such as nitinol. In general, the distal regions may be formed from a material that is generally less rigid than stainless steel but more rigid than a conventional soft tip, which may be made of polyimide, for example. The distal region of the instrument may be in a relatively straight state or shape while it is introduced into the eye and may then take on a relatively curved state or shape after being introduced into the eye. For example, the temperature within the eye (e.g., body temperature around 25-37 degrees Celsius) may cause the distal tip of the instrument to return to a desired state, such as for example, a curved state, formed during fabrication of the device.

The present disclosure may also include methods of using such exemplary instruments to remove subretinal fluid material safely from the surface of the retina. For example, during an ophthalmic operation, such as a vitreoretinal operation, the distal tip may be placed in contact with the retina and activated to aspirate material from the surface of the retina, off of the retina, and out of the vitreous chamber of the eye. For example, during an operation, droplets of silicone oil, perfluorocarbon, blood products, or other droplets may be deposited on the retinal surface. These may be removed from the surface of the retina using the systems, methods, and instruments disclosed herein. The orientation of the side port may prevent the retina from being drawn into the side port, thereby preventing or minimizing the likelihood of harm to the retina itself.

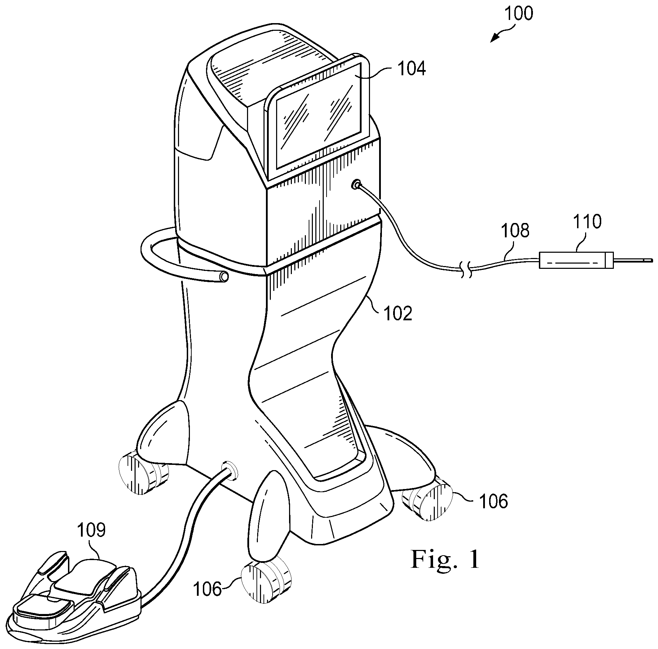

FIG. 1 illustrates an exemplary implementation of an ophthalmic surgical system, generally designated as surgical system 100. While the present disclosure applies to many different types of surgical systems other than the exemplary ophthalmic surgical system 100, the surgical system 100 is described herein to provide appropriate context for the instruments, systems, and methods described herein. As illustrated, the surgical system 100 includes a base housing or console 102 and an associated display screen 104 that may be used to show data relating to system operation and performance during an ophthalmic surgical procedure. In some implementations, the console 102 may be mobile. For example, some implementations may include wheels or casters 106 to facilitate movement about an operating room. In some implementations, the console 102 may not include wheels. The console 102 may contain several subsystems that cooperate to enable a surgeon or other user to perform a variety of surgical procedures, such as ophthalmic surgical procedures.

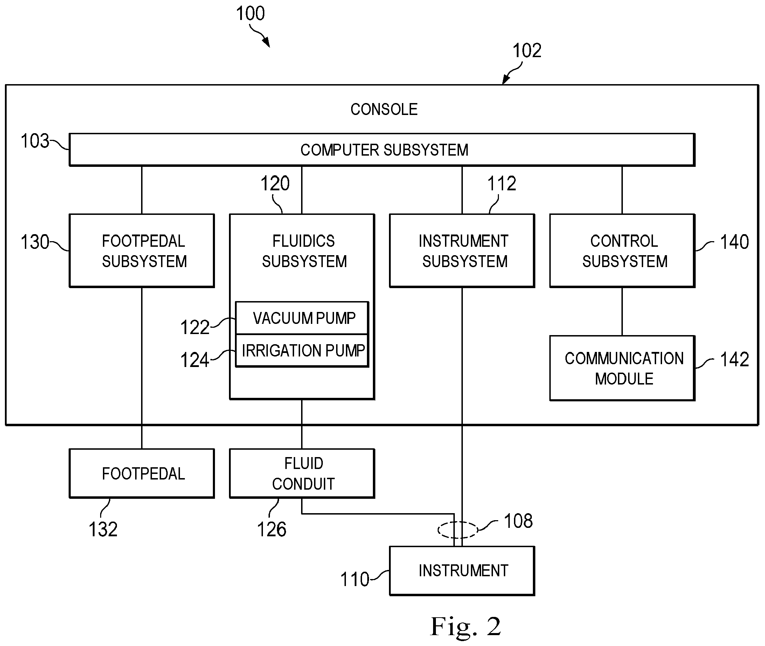

An exemplary surgical instrument, which is illustrated as an instrument 110, may be coupled to the console 102 by a conduit 108 and may form a part of the surgical system 100. Embodiments of the surgical system 100 may include more than one instrument 110. The instrument 110 represents any number of medical and/or surgical instruments, including, for example, a vitrectomy probe, an illumination probe, an aspiration probe, an irrigation probe, a drainage cannula, a phacoemulsification device, a diathermy probe, or other types of medical instruments. The instrument 110 may be a handpiece, in some implementations, such that instrument 110 is configured to be held comfortably in a user's hand for manipulation thereby. The instrument 110 may be coupled to one or more subsystems included in the console 102. For example, the instrument 110 may be coupled to a fluidics subsystem 120 (see FIG. 2) that facilitates control of a pump and/or a vacuum for use in the removal of materials, such as subretinal fluid, from the posterior segment of an eye. In some embodiments, an instrument subsystem 112 (see FIG. 2) may also provide power to the instrument 110 and/or control operation of the instrument 110. The conduit 108 may include cables, tubes, wires, fibers, or conductors, among other carriers, to provide for the operation of the instrument 110. Some implementations may further include a footpedal 109 which can be manipulated by a user to control various aspects of the surgical system 100, including operational parameters, such as flow rates, speeds, irrigation or aspiration, and other parameters of the instrument 110.

As illustrated in FIG. 1, the instrument 110 may be a drainage cannula that may be used in any of a variety of ophthalmic procedures, such as an anterior segment procedure, a posterior segment procedure, a vitreoretinal procedure, a vitrectomy procedure, a cataract procedure, and/or other procedures to drain fluid from the eye. Surgical procedures other than these ophthalmic procedures may be performed by the system 100 and the instrument 110.

FIG. 2 is a block diagram according to an example implementation of the surgical system 100. The surgical system 100 may include the console 102 and several subsystems contained therein. In this example, the console 102 includes a computer subsystem 103 configured to communicate with the display screen 104 (shown in FIG. 1) and with a number of subsystems that are used together to perform ophthalmic surgical procedures, such as vitreoretinal surgical procedures, for example. The computer subsystem 103 may include one or more processing devices, such as a central processing unit or central processor, and a data storage system. The data storage system may include one or more types of memory, such as RAM, ROM, flash memory, a disk-based hard drive, and/or a solid-state hard drive. The processing devices and data storage system may communicate over a bus, which may also permit communication with and between one or more of the subsystems of the surgical system 100.

Some examples of subsystems in the implementation shown in FIG. 2 may include the instrument subsystem 112, the fluidics subsystem 120, and a footpedal subsystem 130 including, for example, the footpedal 109. The fluidics subsystem 120 may provide an aspiration pressure source and an irrigation pressure source. For example, in the implementation shown, the fluidics subsystem 120 includes a vacuum pump 122 and/or an irrigation pump 124. The instrument 110 may be connected to the fluidics subsystem 120 via a fluid conduit 126. In some instances, an instrument connected to the console 120 may be connected to one of the vacuum pump 122 or irrigation pump 124. In other instances, an instrument connected to the console 120 may be connected to both the vacuum pump 122 and the irrigation pump 124 via respective conduits. All or a portion of the one or more fluid conduits connecting the instrument 110 to the console 120, such as the fluid conduit 126, may extend through the conduit 108 (FIG. 1). The surgical system 100 may further include a control subsystem 140 that is coupled to a communication module 142. The control subsystem 140 and the communication module 142 may facilitate control of the instrument 110 and/or the subsystems and other features illustrated in FIG. 2, such as control of the vacuum pump 122 and the irrigation pump 124 of the fluidics subsystem 120.

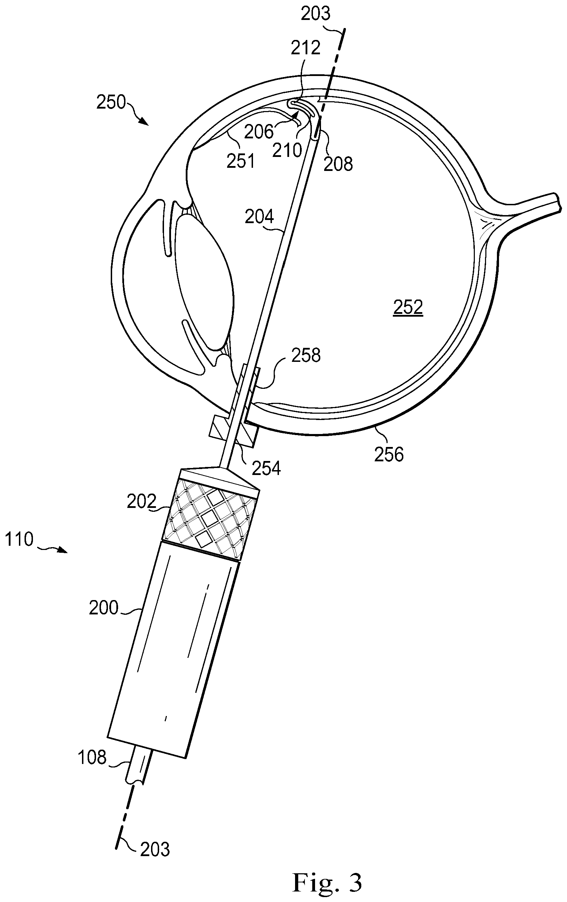

FIG. 3 shows an exemplary instrument 110 inserted into an eye 250. The instrument 110, which may be referred to as a drainage cannula, includes a handle 200 having a proximal end coupled to the conduit 108 (FIG. 1). The handle 200 may include a rotational structure 202 at a distal region of the handle 200. The rotational structure 202 may rotate about a longitudinal axis 203 of the handle 200 and may have a textured or knurled surface to facilitate gripping and rotational movement by a user. The rotational structure 202 may be coupled to a first elongate tubular member 204 that extends distally from the body of the handle 200. The first elongate tubular member 204 has a lumen extending therethrough. The lumen connects to the conduit 108 so as to be coupled to the fluidics subsystem 120, as shown in FIG. 2.

A distal end of the first elongate tubular member 204 is coupled to a second elongate tubular member 208. As shown, the second elongate tubular member 208 may be curved with a curvature that substantially corresponds to the radius of curvature of the interior of the eye 250. In some instances, the radius of curvature may be around 12 mm. A 12 mm radius of curvature may correspond to the curvature of an eye of adults. In some embodiments, a portion of the second elongate tubular member 208 may have a curvature substantially corresponding to the radius of curvature of the eye 250, while another portion of the second elongate tubular member 208 may have a curvature that does not substantially correspond to the radius of curvature of the eye 250. The second elongate tubular member 208 may include an opening or port 210 formed through a sidewall of the elongate tubular member 208 that provides access to a lumen 212 extending within the second elongate tubular member 208 and connecting to the lumen of the elongate tubular member 204 so as to form a continuous lumen. In some implementations, the port 210 may have an oblong shape or elliptical shape and may have a smoothed edge to prevent damage to the retina. The port 210 may be an oblong or elongate port having a longitudinal or major axis extending longitudinally along a portion of the tubular member 208.

In some embodiments, the port 210 may be a collection of smaller ports that function collectively to drain subretinal fluid at one location of the second elongate tubular member 208. The port 210 may be sized such that the port 210 occupies less than a total of 90 degrees of the circumference of the second elongate tubular member 208, in some implementations. Other implementations, the port 210 may be sized such that the port 210 occupies more or less of the circumference of the second elongate tubular member 208. The port 210 may be the only port formed in the second elongate tubular member 208 around the circumference thereof. That is, the second elongate tubular member 208 may include a single port, i.e., port 210. The distal tip 214 of the second elongate tubular member 208 may be capped and rounded to avoid snagging or otherwise damaging the retina 251 or RPE of the eye 250.

The first and second elongate tubular members 204 and 208 may be coupled to the rotational structure 202 such that rotation of the rotational structure 202 may simultaneously cause the first and second elongate tubular members 204 and 208 to rotate as well. For example, the first and second elongate tubular members 204 and 208 may be fixed to the rotational structure 202 so that the first elongate tubular member 204, the second elongate tubular member 208, and the rotational structure 202 all rotate together. In some implementations, the first and second elongate tubular members 204 and 208 may be formed from different materials that have different material properties. For example and without limitation, the first elongate tubular member 204 may be formed from stainless steel, while the second elongate tubular member 208 may be formed from nitinol or another shape memory alloy. In some implementations, the first elongate tubular member 204 may generally be more rigid than the second elongate tubular member 208. In some implementations, the second elongate tubular member 208 may be made from the shape memory alloy or a deformable polymeric material that is more rigid than silicone. More generally, the second elongate tubular member 208 may be formed from a material that may be deformed temporarily and then return to an original shape without application of an external force.

A benefit of the rigidity of the second elongate tubular member 208, particularly a rigidity that is greater than one associated with soft-tipped instruments, is that the second elongate tubular member 208 is less prone to buckle or bend under pressures required to remove subretinal fluid. As a consequence, a risk associated with deflection of the second elongate tubular member 208 to one side or another during a surgical procedure, which may otherwise cause incarceration of the retina in the port 210, may be substantially reduced or eliminated. This is an important benefit as uncontrolled buckling of an instrument towards the retina can result in incarceration of the retina, e.g., via a port formed in the instrument, or otherwise contact and injure the retina.

A distal region 206 of the instrument 110, which includes the second elongate tubular member 208, may be positioned within the vitreous chamber 252 of the eye 250 by passing through a trocar cannula 254 (shown in cross-section in FIG. 3). The trocar cannula 254 may be used to form and maintain an opening through the sclera 256. In some implementations, the trocar cannula 254 may have an interior diameter of up to 1 mm. The first and second elongate tubular members 204 and 208 may have an outer diameter ranging from about 0.3 mm to about 0.7 mm. In some implementations, the outer diameter of both the first and second elongate tubular members 204 and 208 are the same. In other implementations, the outer diameter of first and second elongate tubular members 204 and 208 may be different. For example, in some implementations, the first elongate tubular member 204 may be a 25 gauge needle, with an outer diameter less than 0.55 mm. In such implementations, the first and second elongate tubular members 204 and 208 may both have an outer diameter less than 0.55 mm. Further, the outer diameters of the first elongate tubular member 204 and the second elongate tubular member 208 may be substantially the same, and, in some implementations, the first and second elongate tubular members 204 and 208 may abut each other and be fixedly coupled together.

In operation, the curved second elongate tubular member may be straightened such that the second elongate tubular member 208 is longitudinally straight and aligned with the first elongate tubular member 204. Altering the shape of the second elongate tubular member 208 in this manner provides for ease in passing the first and second elongate tubular members 204 and 208through a lumen 258 of the trocar cannula 254. In some implementations, the lumen 258 may be a cylindrical lumen. After the tubular member 208 has passed through the lumen 258, tubular member 208 may return to its curved shape or state without application of external force. For example, some implementations of the second elongate tubular member 208 are deformable and biased toward a curved state. However, in such implementations, the second elongate tubular members 208 may be deformable into a straight shape to facilitate passing the second elongate tubular member 208 through the lumen 258 of the trocar cannula 254 and into the eye 250 of a patient. In still other implementations, once placed into a straightened shape, the second elongate tubular member 208 may return to a curved shape due to a change in temperature of the second elongate tubular member 208 once inserted into the eye 250. For example, once inserted into the eye, the second elongate tubular member 208 may be warmed by the eye 250, thereby raising a temperature of the elongate tubular member 208 above a transition temperature at which the second elongate tubular member 208 returns to its initial, curved shape.

For example, the tubular member 208 may be formed from nitinol or another material having a shape that is affected or controllable by temperature. The temperature at which the tubular member 208 transitions from a relatively straight state to a relatively curved state may be controlled by the proportions of nickel and titanium used in the alloy and/or by the annealing temperature used to form the tubular member 208. As noted herein, other implementations of the tubular member 208 may be formed from other shape memory alloys or other shape memory polymers.

For example, the tubular member 208 may be straight or relatively straighter when at the ambient temperature of an operating room environment. After the tubular member 208 passes through the lumen 258 of the trocar cannula 254, the exposure to body temperature within the eye 250 may cause the tubular member 208 to assume a predetermined shape or curvature.

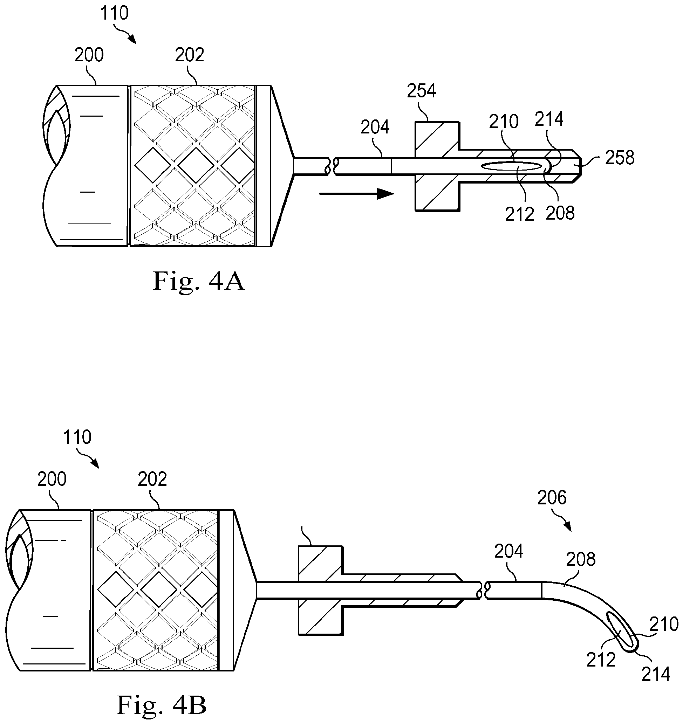

FIGS. 4A and 4B illustrate an implementation of the instrument 110. In FIG. 4A, the instrument 110 is shown partially inserted through the central lumen 258 of the trocar cannula 254. The second elongate tubular member 208 is illustrated in a straightened state. This straightened state may be provided by the physical constraints of the central lumen 258 and/or by the physical properties, of the material from which the second elongate tubular member 208 is formed, e.g., temperature sensitivity associated with shape-memory materials that change shape as a result of changes in temperature. Fig. 4B depicts the instrument 110 as being inserted further through the trocar cannula 254 such that a portion of the first elongate tubular member 204 and the second elongate tubular member 208 extend beyond the trocar cannula 254. For example, the first and second elongate tubular members 204 and 208 may protrude into the vitreous chamber 252 of the eye 250, shown in FIG. 3. Either due to the lack of constraint imposed by the walls of the lumen 258 of the trocar cannula 254 or due to the ambient temperature within the eye 250 (or a combination thereof), the second elongate tubular member 208 assumes a curved shape. The curvature of the second elongate tubular member 208 may correspond at least in part to the curvature of the eye 250. The shape of the port 210 may change according to the straightened or curved state of the elongate tubular member 208.

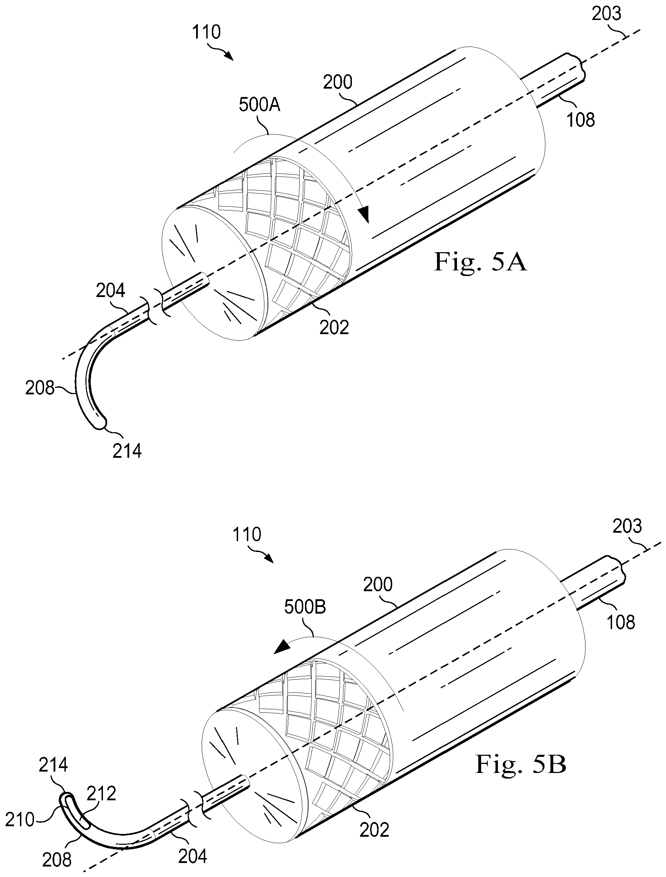

Referring now to FIGS. 5A and 5B, as shown therein, a user may manipulate the rotational structure 202 in order to rotate the first and second elongate tubular members 204 and 208 about the longitudinal axis 203. For example, the user may desire to reorient the port 210 for insertion through a tear or opening in the retina 251 in order to drain subretinal fluid so that the retina 251 may be repositioned and reattached to the RPE/choroid. As shown in FIG. 5A, the user may rotate the rotational structure 202 according to the arrow 500A. By rotating the rotational structure 202 by approximately 90°, the second elongate tubular member 208 may be repositioned as seen in FIG. 5B, such that the port 210 is in view. By moving the rotational structure 202 according to the arrow 500B, the user may further adjust the positioning of the port 210 within the eye 250. Thus, in some implementations, the rotational structure 202 and, hence, the first and second elongate tubular members 204 and 208 may be rotated about the longitudinal axis 203 in either of the directions corresponding to arrows 500A and 500B.

While some implementations of the instrument 110 may have a limited range of rotation, some implementations of the instrument 110 may permit the rotational structure 202 to rotate freely in either direction (clockwise or counterclockwise). Unrestricted rotation of the rotational structure may allow a user, for example, to avoid incarcerating the retina within the port 210 when draining subretinal fluid. The user may turn the rotational structure 202 to align the distal tip of the second elongate tubular member 208 with an opening in the retina. The user may also turn the rotational structure 202 in order to conform the curvature of the second elongate tubular member 208 to the curvature of the eye 250 in order to remove material from the surface of the retina, for example.

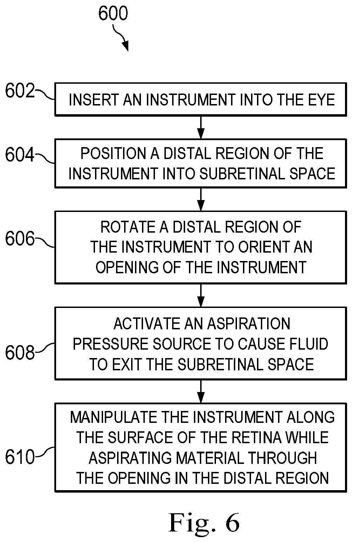

FIG. 6 is a flowchart of an example method 600 for utilizing an instrument, such as a drainage cannula which may be similar to instrument 110, to remove subretinal fluid and/or material from a delicate surface, like the surface of the retina of the eye, such as the eye 250 shown in FIG. 3. Method 600 is illustrated in FIG. 6 as several enumerated operations or steps. Implementations of the method 600 may include additional operations, before, after, in between, or as sub-operations of the enumerated operations. Additionally, some implementations of the method 600 may omit one or more of the enumerated operations.

At 602, an instrument, such as a drainage cannula similar to the instrument 110 of FIGS. 1-3, 4A, 4B, 5A, and 5B, may be introduced into the eye of a patient. The instrument may include a distal region with a port, which may be similar to the port 210. At least a portion of the instrument may be inserted through a trocar cannula, which may be similar to the trocar cannula shown in FIG. 3, or through an incision made through the sclera of an eye. A distal region of the instrument may be positioned proximate the retina. In some implementations, the distal region of the instrument is configured to be in a straight state while passing through the incision or trocar cannula. In the case of an incision without a trocar cannula, a straight state of the distal region enables the incision to remain small, minimizing eye trauma. When within the posterior segment of the eye, a portion of the distal region may curve without the application of an external load to correspond to the shape of an average patient's eye. In some of these implementations, the port is disposed on the curved distal region.

At 604, the distal region of the instrument may be positioned into subretinal space by a user. For example, the distal region of the instrument may be inserted through a retinal tear into a space occupied by subretinal fluid, as shown in FIG. 3. To reposition and reattach the retina, the subretinal fluid should be drained. At 606, an aspiration pressure source may be activated to cause fluid to exit the subretinal space. For example, the aspiration pressure source may be provided by the fluidics subsystem 120 shown in FIG. 2. In some implementations, a naturally occurring pressure may cause the subretinal fluid to drain through the instrument. For example, if the pressure within the subretinal space is higher than the pressure within the instrument, the subretinal fluid may flow without the activation of an aspiration pressure source due to the naturally occurring pressure gradient.

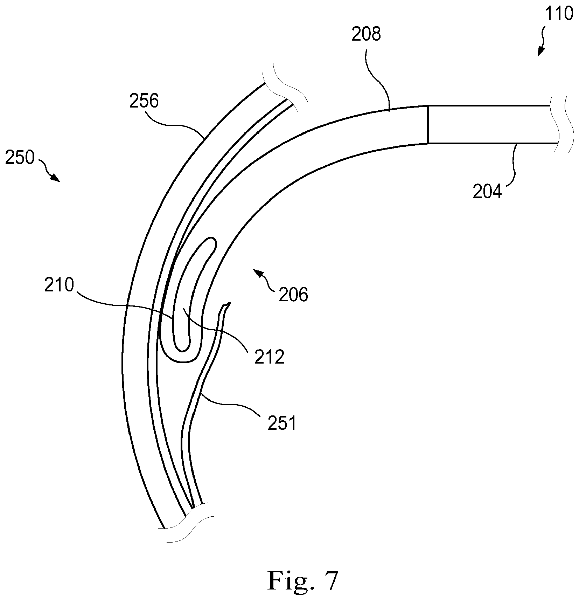

In some implementations, the method 600 may further include 608 and 610. These operations may be performed when the surface of the retina, or other delicate tissue, is to be cleaned. At 608, the distal region of the instrument may be rotated by user manipulation of a rotational structure on a handle body of the instrument. For example, a second elongate tubular member, which may be similar to the second elongate tubular member 208 discussed herein, may be rotated as the rotational structure is manipulated by a user. Similarly, the rotational structure may be similar to the rotational structure 202 described herein. The second elongate tubular member may be rotated such that the port 210 is oriented perpendicular to a desired cleaning path. This orientation is illustrated in FIG. 7. As shown in FIG. 7, the first and second elongate tubular members 204, 208 are oriented such that the port 210 is substantially orthogonally to the retina 251. "Substantially" is utilized here to describe the relative position of the port 251 relative to the retina 251 as both the retina 251 has a curved shape and the port 251 is formed in the second elongate tubular member 208 that also, in the implementation shown, has a tubular shape. The instrument 110 may be moved relative to the eye 250, such as in directions into an out of the plane of the drawing and along the curvature of the eye 250 shown in FIG. 7. The orientation of the port 210 in the manner shown and described and movement of the instrument 110 relative to the eye as described reduces the risk of incarcerating the retina 251 within the port while removing subretinal fluid. As a result, injury to the retina is reduced.

In some instances, if the distal region of the instrument is oriented as desired upon insertion, it may not be necessary for the user to manipulate the instrument to rotate the second elongate tubular member as part of the method 600. At 610, the portion of the instrument outside the eye may be manipulated so that the second elongate tubular member travels along the retina while a pressure gradient is used to aspirate material from the eye. The instrument may work analogously to a vacuum cleaner; however, the opening of the instrument (e.g., the port in the tubular member, which may be similar to port 210 described herein) may be oriented orthogonally to or away from the surface of the retina, not oriented toward the retina. In this way, the instrument is prevented from grasping the retina itself, avoiding potential harm to the retina. Accordingly, the instrument 110 may be understood and used as a cleaning instrument configured to clean the surface of the retina.

The instruments, systems, and methods described herein enable a user to remove retinal fluids from between a retina and a choroid, helping retinal tissue maintain contact with the choroid after a retinal tear or detachment. As such, the retinal tissue may receive nourishment from the blood vessels within the eye and may begin to heal in place, providing a satisfactory outcome for a patient.

Persons of ordinary skill in the art will appreciate that the implementations encompassed by the present disclosure are not limited to the particular exemplary implementations described above. In that regard, although illustrative implementations have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed in a manner consistent with the present disclosure.

## Claims

1. A surgical instrument (110) for removal of subretinal fluid, the surgical instrument comprising:
a handle (200) coupleable to an aspiration pressure source, the handle having a rotational structure (202) that is rotatable around an axis (203) of a handle body when manipulated by a user;
a first elongate tubular member (204) having a first proximal end and a first distal end, the first proximal end being coupled to the rotational structure such that the first elongate tubular member is rotatable around the axis;
a second elongate tubular member (208) having a second proximal end and a second distal end, the second proximal end of the second elongate tubular member being coupled to the first distal end of the first elongate tubular member, and wherein the second elongate tubular member is curved when exposed to body temperature; and
a port (210) formed through a wall of the second elongate tubular member (208) for aspirating material from a body cavity through the first and second elongate tubular members.

2. The surgical instrument of claim 1 wherein the second elongate tubular member (208) is formed from a shape memory alloy.

3. The surgical instrument of claim 2, wherein the shape memory alloy is nitinol.

4. The surgical instrument of claim 1, wherein the first elongate tubular member (204) is straight.

5. The surgical instrument of claim 1, wherein the port (210) is an oblong shaped port that includes a smoothed edge.

6. The surgical instrument of claim 1, wherein the second distal end of the second elongate tubular member (208) is a closed and rounded distal tip.

7. The surgical instrument of claim 1, wherein the second elongate tubular member (208) is substantially straight when exposed to a temperature below body temperature.

8. The surgical instrument of claim 1, wherein a curve of the second elongate tubular member (208) corresponds to curvature of a human eye.

9. The surgical instrument of claim 1, wherein the first elongate tubular member (204) is formed from a first material and the second elongate tubular member (208) is formed from a second material, wherein the first material is more rigid than the second material.

10. The surgical instrument of claim 1, wherein the second elongate tubular member (208) has an outer diameter ranging from about 0.3 mm to about 0.7 mm.

11. The surgical instrument of claim 1, wherein :
the first elongate tubular member (204) further comprises a first lumen extending through the first elongate tubular member;
the second elongate tubular member (208) further comprises
a second lumen extending through the second elongate tubular member,
wherein the first lumen is in fluid communication with the second lumen; and
the port (210) is formed on one side of the second elongate tubular member, the port configured to aspirate the material from the body cavity through the first lumen and the second lumen.

12. The surgical instrument of claim 1, wherein the second elongate tubular member is formed from a shape memory alloy and has a curved shape.

13. The surgical instrument of claim 1, wherein the first elongate tubular member (204) is formed from stainless steel and wherein the second elongate tubular member (208) is less rigid than the first elongate tubular member.

14. The surgical instrument of claim 1, wherein the port (210) has an elliptical shape.

15. The surgical instrument of claim 1, wherein the port (210) is the only port formed in the second elongate tubular member.

## Patentansprüche

1. Chirurgisches Instrument (110) zur Entfernung von subretinaler Flüssigkeit, wobei das chirurgische Instrument Folgendes umfasst:
einen an eine Aspirationsdruckquelle koppelbaren Griff (200), wobei der Griff eine Drehstruktur (202) hat, die bei Handhabung durch einen Benutzer um eine Achse (203) eines Griffkörpers drehbar ist,
ein erstes längliches röhrenförmiges Glied (204) mit einem ersten proximalen Ende und einem ersten distalen Ende, wobei das erste proximale Ende so an die Drehstruktur gekoppelt ist, dass das erste längliche röhrenförmige Glied um die Achse drehbar ist,
ein zweites längliches röhrenförmiges Glied (208) mit einem zweiten proximalen Ende und einem zweiten distalen Ende, wobei das zweite proximale Ende des zweiten länglichen röhrenförmigen Glieds an das erste distale Ende des ersten länglichen röhrenförmigena Glieds gekoppelt ist und wobei das zweite längliche röhrenförmige Glied gekrümmt ist, wenn es Körpertemperatur ausgesetzt ist,
und
eine Öffnung (210), die durch eine Wand des zweiten länglichen röhrenförmigen Glieds (208) ausgebildet ist, um Material aus einer Körperhöhle durch das erste und das zweite längliche röhrenförmige Glied zu aspirieren.

2. Chirurgisches Instrument nach Anspruch 1, wobei das zweite längliche röhrenförmige Glied (208) aus einer Formgedächtnislegierung ausgebildet ist.

3. Chirurgisches Instrument nach Anspruch 2, wobei die Formgedächtnislegierung Nitinol ist.

4. Chirurgisches Instrument nach Anspruch 1, wobei das erste längliche röhrenförmige Glied (204) gerade ist.

5. Chirurgisches Instrument nach Anspruch 1, wobei die Öffnung (210) eine Öffnung mit länglicher Form ist, die einen geglätteteten Rand aufweist.

6. Chirurgisches Instrument nach Anspruch 1, wobei das zweite distale Ende des zweiten länglichen röhrenförmigen Glieds (208) eine geschlossene und abgerundete distale Spitze ist.

7. Chirurgisches Instrument nach Anspruch 1, wobei das zweite längliche röhrenförmige Glied (208) im Wesentlichen gerade ist, wenn es einer unter der Körpertemperatur liegenden Temperatur ausgesetzt ist.

8. Chirurgisches Instrument nach Anspruch 1, wobei eine Krümmung des zweiten länglichen röhrenförmigen Glieds (208) der Krümmung eines menschlichen Auges entspricht.

9. Chirurgisches Instrument nach Anspruch 1, wobei das erste längliche röhrenförmige Glied (204) aus einem ersten Material ausgebildet ist und das zweite längliche röhrenförmige Glied (208) aus einem zweiten Material ausgebildet ist, wobei das erste Material starrer als das zweite Material ist.

10. Chirurgisches Instrument nach Anspruch 1, wobei das zweite längliche röhrenförmige Glied (208) einen äußeren Durchmesser hat, der sich zwischen ungefähr 0,3 mm und ungefähr 0,7 mm bewegt.

11. Chirurgisches Instrument nach Anspruch 1, wobei:
das erste längliche röhrenförmige Glied (204) ferner Folgendes umfasst:
ein sich durch das erste längliche röhrenförmige Glied erstreckendes erstes Lumen,
das zweite längliche röhrenförmige Glied (208) ferner Folgendes umfasst:
ein sich durch das zweite längliche röhrenförmige Glied erstreckendes zweites Lumen,
wobei das erste Lumen in Fluidverbindung mit dem zweiten Lumen steht,
und
die Öffnung (210) an einer Seite des zweiten länglichen röhrenförmigen Glieds ausgebildet ist, wobei die Öffnung dazu ausgestaltet ist, das Material aus der Körperhöhle durch das erste Lumen und das zweite Lumen zu aspirieren.

12. Chirurgisches Instrument nach Anspruch 1, wobei das zweite längliche röhrenförmige Glied aus einer Formgedächtnislegierung ausgebildet ist und eine gekrümmte Form hat.

13. Chirurgisches Instrument nach Anspruch 1, wobei das erste längliche röhrenförmige Glied (204) aus Edelstahl ausgebildet ist und das zweite längliche röhrenförmige Glied (208) weniger starr als das erste längliche röhrenförmige Glied ist.

14. Chirurgisches Instrument nach Anspruch 1, wobei die Öffnung (210) eine elliptische Form hat.

15. Chirurgisches Instrument nach Anspruch 1, wobei die Öffnung (210) die einzige in dem zweiten länglichen röhrenförmigen Glied ausgebildete Öffnung ist.

## Revendications

1. Instrument chirurgical (110) pour l'élimination d'un liquide sous-rétinien, l'instrument chirurgical comprenant :
un manche (200) pouvant être accouplé à une source de pression d'aspiration, le manche ayant une structure rotative (202) qui peut tourner autour d'un axe (203) d'un corps de manche lorsqu'il est manipulé par un utilisateur ;
un premier élément tubulaire allongé (204) ayant une première extrémité proximale et une première extrémité distale, la première extrémité proximale étant accouplée à la structure rotative de sorte que le premier élément tubulaire allongé puisse tourner autour de l'axe ;
un second élément tubulaire allongé (208) ayant une seconde extrémité proximale et une seconde extrémité distale, la seconde extrémité proximale du second élément tubulaire allongé étant accouplée à la première extrémité distale du premier élément tubulaire allongé, et dans lequel le second élément tubulaire allongé est courbé lorsqu'il est exposé à la température corporelle ; et
un orifice (210) formé à travers une paroi du second élément tubulaire allongé (208) pour aspirer un matériau provenant d'une cavité corporelle à travers les premier et second éléments tubulaires allongés.

2. Instrument chirurgical selon la revendication 1, dans lequel le second élément allongé tubulaire (208) est en alliage à mémoire de forme.

3. Instrument chirurgical selon la revendication 2, dans lequel l'alliage à mémoire de forme est le nitinol.

4. Instrument chirurgical selon la revendication 1, dans lequel le premier élément allongé tubulaire (204) est rectiligne.

5. Instrument chirurgical selon la revendication 1, dans lequel l'orifice (210) est un orifice de forme oblongue qui comprend un bord poli.

6. Instrument chirurgical selon la revendication 1, dans lequel la seconde extrémité distale du second élément tubulaire allongé (208) est une pointe distale fermée et arrondie.

7. Instrument chirurgical selon la revendication 1, dans lequel le second élément tubulaire allongé (208) est sensiblement rectiligne lorsqu'il est exposé à une température inférieure à la température corporelle.

8. Instrument chirurgical selon la revendication 1, dans lequel une courbe du second élément tubulaire allongé (208) correspond à une courbure d'un œil humain.

9. Instrument chirurgical selon la revendication 1, dans lequel le premier élément tubulaire allongé (204) est fait d'un premier matériau et le second élément tubulaire allongé (208) est fait d'un second matériau, dans lequel le premier matériau est plus rigide que le second matériau.

10. Instrument chirurgical selon la revendication 1, dans lequel le second élément tubulaire allongé (208) a un diamètre extérieur dans la plage d'environ 0,3 mm à environ 0,7 mm.

11. Instrument chirurgical selon la revendication 1, dans lequel :
le premier élément tubulaire allongé (204) comprend en outre
une première lumière s'étendant à travers le premier élément tubulaire allongé ;
le second élément tubulaire allongé (208) comprend en outre
une seconde lumière s'étendant à travers le second élément tubulaire allongé,
la première lumière étant en communication fluidique avec la seconde lumière ; et
l'orifice (210) est formé sur un côté du second élément tubulaire allongé, l'orifice étant conçu pour aspirer le matériau provenant de la cavité corporelle à travers la première lumière et la seconde lumière.

12. Instrument chirurgical selon la revendication 1, dans lequel le second élément tubulaire allongé est en alliage à mémoire de forme et a une forme courbée.

13. Instrument chirurgical selon la revendication 1, dans lequel le premier élément tubulaire allongé (204) est en acier inoxydable et dans lequel le second élément tubulaire allongé (208) est moins rigide que le premier élément tubulaire allongé.

14. Instrument chirurgical selon la revendication 1, dans lequel l'orifice (210) a une forme elliptique.

15. Instrument chirurgical selon la revendication 1, dans lequel l'orifice (210) est le seul orifice formé dans le second élément tubulaire allongé.
